(19) **Europäisches Patentamt**

**European Patent Office**

**Office européen des brevets**

(11) **EP 0 826 666 A1**

(12) **EUROPEAN PATENT APPLICATION**

(43) Date of publication:
**04.03.1998 Bulletin 1998/10**

(51) Int. Cl.⁶: $C07D\ 201/00$, $C07D\ 201/08$

(21) Application number: **96202438.6**

(22) Date of filing: **02.09.1996**

(84) Designated Contracting States:
**AT BE CH DE DK ES FI FR GB GR IE IT LI LU MC NL PT SE**

(71) Applicant: **DSM N.V.**
**6411 TE Heerlen (NL)**

(72) Inventors:
- **Guit, Rudolf Philippus Maria**
  **6228 GP Maastricht (NL)**
- **Buijs, Wim**
  **6365 BM Schinnen (NL)**

(54) **Process to prepare epsilon-caprolactam**

(57) Process to prepare ε-caprolactam, in which in a step (a) a compound with the general formula:

$$O{=}CH{-}(CH_2)_4{-}C(O){-}R \qquad (1)$$

in which R is -OH, $-NH_2$ or O-R', in which R' is an organic group with 1 to 10 carbon atoms, is contacted with ammonia and hydrogen in a suitable solvent at elevated pressure in the presence of a hydrogenation catalyst to a mixture of primary amino compounds and ε-caprolactam, followed by a separate second step (b) in which the primary amino compounds are reacted to ε-caprolactam, wherein the solvent in step (a) is water or an aqueous medium, the yield to ε-caprolactam in step (a) is more than 10%, calculated on the initial molar amount of the compound according to formula (1), that ε-caprolactam is separated from the aqueous mixture obtained in step (a) by extraction and that the aqueous mixture resulting from the extraction, containing the primary amino compounds, is used in step (b).

Printed by Xerox (UK) Business Services
2.15.11/3.4

## Description

The invention relates to a process to prepare ε-caprolactam, in which in a step (a) a compound with the general formula:

$$O=CH-(CH_2)_4-C(O)-R \qquad (1)$$

in which R is -OH, -NH$_2$ or -OR', in which R' is an organic group with 1 to 10 carbon atoms, is contacted with ammonia and hydrogen in a suitable solvent at elevated pressure in the presence of a hydrogenation catalyst to a mixture of primary amino compounds and ε-caprolactam, followed by a separate second step (b) in which the primary amino compounds are reacted to ε-caprolactam.

Such a process is described in US-A-4730041. This patent publication describes a process in which first methyl 5-formylvalerate is reacted with excess ammonia and hydrogen in the pressence of methanol as solvent and in the pressence of a catalyst, such as Raney-nickel, in the liquid phase at 80°C to yield 89% methyl 6-aminocaproate and about 3% ε-caprolactam. This mixture is subsequently heated to 225°C to yield 78% ε-caprolactam. The total concentration of all of the reactants in the different process steps was about 10 wt%.

A disadvantage of the process described in US-A-4730041 is that relatively large process equipment for the cyclization section (step b) is required. This is because of the low concentration of reactants in that step. According to US-A-4730041 the cyclization is performed at super atmospheric pressures, requiring special process equipment. From an economical/investment point of view smaller process equipment is desired. Using smaller process equipment by increasing the concentration in the cyclization is according to an article of Mares and Sheehan in Ind. Eng. Chem. Process Des. Dev., Vol. 17, No. 1, 1978, 9-16, not advantageous because yield loss is to be expected due to the formation of oligomers.

The aim of this invention is a process which requires a smaller volume of process equipment for the cyclization section (step b) compared to a state of the art process.

This aim is achieved in that the solvent in step (a) is water or an aqueous medium, the yield to ε-caprolactam in step (a) is more than 10%, calculated on the initial molar amount of the compound according to formula (1), that ε-caprolactam is separated from the aqueous mixture obtained in step (a) by extraction using an organic extraction agent and that the aqueous mixture resulting from the extraction, containing the primary amino compound, is used in step (b).

By using water as the solvent in step (a) and increasing the ε-caprolactam yield in step (a), ε-caprolactam can be advantageously be separated from the reaction mixture prior to step (b) by extraction. By separating part of the ε-caprolactam prior to step (b) a smaller volume of process equipment can be used in step (b).

A further advantage is that part of the ε-caprolactam is prepared at a relatively low temperature of the first step. In the process of US-A-4730041 almost all of the ε-caprolactam is prepared in the second step at relatively high temperatures, for example 300°C. This difference will result in that the overall energy consumption to prepare one mol of ε-caprolactam of the process according to the invention will be less than of the state of the art process. The fact that less ε-caprolactam is exposed to the higher temperature of the second step is also advantageous for the level of impurities in the ε-caprolactam thus obtained. At higher temperatures ε-caprolactam tends to react to impurities more easily than at a lower temperature level.

Another advantage is that higher overal yields to ε-caprolactam can be obtained than was possible with the state of the art process as described in US-A-4730041.

Examples of processes which yield more than 10% in step (a) are not reported in the prior art, probably because at higher yields to ε-caprolactam oligomers can be formed. Oligomer formation is as a rule considered to be disadvantageous for the yield to ε-caprolactam. We have found that such oligomer formation in step (a) does not have to result in a reduction of the overall ε-caprolactam yield.

The extraction of ε-caprolactam from the aqueous mixture can be performed with any organic extraction solvent which is immiscible with the aqueous mixture.

Examples of such solvents are ethers, for example methyl tert-butylether, aromatics, for example toluene, benzene and xylene and parafinic solvents, for example decaline. Preferably chlorinated hydrocarbons with 1 to 10 carbon atoms are used. Examples are dichloromethane, chloroform, 1,1,1-trichloroethane. Examples of another class of extraction agents are phenol and alkyl phenols. A preferred class of alkyl phenols have a boiling point which is higher than that of ε-caprolactam. It has been found that ε-caprolactam can be separated by extraction from the amine compounds obtained in step (a).

The alkyl phenol has peferably a boiling point higher than the boiling point of ε-caprolactam, which is 270°C at 0.1 MPa. Alkyl phenols have a high boiling point at atmospheric pressure. Therefore, boiling points are advantageously compared at reduced pressures of, for example, 1.3 kPa (10 mmHg). Caprolactam has a boiling point of 140°C at 10 mmHg, while dodecyl phenol, for example, has a boiling point of 190°C at that pressure. By preference, the boiling point of the alkyl phenol is more than about 5°C, and in particular, more than about 15°C higher than the caprolactam boiling point at 1.3 kPa (10 mmHg). The upper limit to the boiling point of the alkyl phenol is about 400°C. The alkyl phenol preferably is non-azeotropic with ε-caprolactam. Mixtures of alkyl phenols can also be used.

An alkyl phenol is phenol substituted with one or more alkyl groups. The total number of carbon atoms of the alkyl group(s) is preferably between 6-25 and more preferably between 9-15. Examples of specific alkyl phenolic compounds include dodecyl phenol, octyl phenol, nonyl phenol, n-hexyl phenol, 2,4-diisobutyl phenol, 2-methyl-4,6-di-tert-butyl phenol, 3-ethyl-4,6-di-tert-butyl phenol, 2,4,6-tri-tert-butyl phenol, and mixtures of any thereof. US-A-4013640 discloses additional alkyl phenols, the complete disclosure of which is hereby incorporated by reference.

The extraction step is, carried out at a temperature which is higher than the melting point of the organic extraction agent. The temperature of extraction can be generally between room temperature and 200°C.

The pressure during the extraction step is not critical and can be, for example, between about 0.1 MPa and about 2.0 MPa, and preferably, between about 0.1 MPa and about 0.5 MPa.

The extraction can be carried out in well known extraction apparatus, for example a counter current column or a series of mixer/settlers. The extraction step yields a ε-caprolactam-containing organic phase which, which in general, contains up to 50 wt.% ε-caprolactam and between about 0 and about 15 wt.% water. Preferably extraction agents are used in which water will not dissolve significantly.

It has been found that ε-caprolactam can be exclusively separated from an aqueous mixture containing 6-aminocaproic acid, 6-aminocaproamide and/or oligomers. These primary amino compounds are the most important reaction products of step (a) and the starting compounds of the reaction to ε-caprolactam in step (b).

The starting compound according to formula (1) (the aldehyde compound) can be obtained by hydroformylation of the corresponding pentenoate ester, acid or amide as for example described for the ester in WO-A-9426688 and WO-A-9518089 and for the acid in for example WO-A-9518783. Preference is given to the 5-formylvalerate ester as starting compound because this compound is more easily obtainable.

R in formula (1) is a -OH, -NH$_2$ or -O-R' group, in which R' is preferably an organic group with 1 to 20 carbon atoms, wherein the organic group is an alkyl, cycloalkyl, aryl or aralkyl group. More preferably R' is an alkyl group. Examples of R' groups include methyl, ethyl, propyl, isopropyl, n-butyl, tert-butyl, isobutyl, cyclohexyl, benzyl and phenyl. By preference R' is methyl or ethyl.

Step (a) can be performed by the generally known methods for reductive amination. When starting from 5-formylvaleric acid the conditions as described in for example US-A-4730040 can be applied and when starting from a 5-formylvalerate ester the conditions as described in US-A-4730041 can be applied. Ammonia is preferably present in a molar excess to the aldehyde compound. When starting from a 5-formylvalerate ester it is preferred to perform the step (a) in the presence of an additional alcohol solvent and more preferably in the presence of the corresponding alcohol of the ester (R'-OH). The presence of the alcohol improves the solubility of the 5-formylvalerate ester in the aqueous reaction mixture. The concentration of the alcohol is preferably between 2 and 20 wt.% and more preferably between 5 and 15 wt.%. The temperature in step (a) is preferably between 50 and 150°C and the pressure is between 0,5 and 20 MPa.

The hydrogenation catalyst comprises one or more of the metals choosen from the metals of groups 8-10 of the Periodic System of the Elements (New IUPAC notation; Handbook of Chemistry and Physics, 70th edition, CRC Press, 1989-1990) for example nickel, cobalt, ruthenium, platinum or palladium. Preference is given to Ru-, Ni- or Co-containing catalysts. In addition to Ru, Co and/or Ni the catalysts can also contain other metals for example Cu, Fe and/or Cr. The content of these additional metals may, for example, be up to 20% by weight based on the total metal content. The catalytically active metals may be applied onto a carrier or not. Suitable carriers are for example aluminium oxide, silica, titanium oxide, zirconium oxide, magnesium oxide, carbon or graphite. Non-carried metals can also be used. An example of a non-carried metal is finely dispersed ruthenium. Preferred Ni- and Co-containing catalysts are Raney nickel and Raney cobalt optionally in combination with small amounts of another metal, for example Cu, Fe and/or Cr.

Most preferred are ruthenium containing catalysts. High yields to ε-caprolactam in step (a) over a prolonged period of time are possible when using ruthenium containing catalysts. Examples of possible ruthenium containing catalysts are non-carried metal catalysts, for example finely dispersed ruthenium or ruthenium on a carrier, for example ruthenium on a carbon, alumina, graphite or TiO$_2$ carrier.

Step (a) is preferably performed in the below described manner. It has been found that higher overall yields to ε-caprolactam can then be achieved. In this preferred embodiment step (a) is performed in two separate steps (Step (a1) and step (a2)) in which in step (a1) the aldehyde compound according to formula (1) is reacted with ammonia under non-hydrogenating conditions and in step (a2) the reaction product obtained in step (a1) is converted to ε-caprolactam and primary amino compounds under hydrogenating conditions in the presence of ammonia.

Step (a1) is carried out under non-hydrogenating conditions. The term 'non-hydrogenating conditions' means that the reaction conditions are such that, either no hydrogen is present or if hydrogen is present, then the aldehyde compound according to formula (1) or a reaction product of it is not or virtually not reduced by the hydrogen. In general, non-hydrogenating conditions are realized by carrying out the first step in the absence of a hydrogenation catalyst.

In one such embodiment of the present process the hydrogen (needed in step (a2)) can be already present in step (a1). However, if the hydrogenation catalyst is already present in this step, non-hydrogenating conditions are achieved by not adding hydrogen to the reaction mixture until after completing step (a1). A third possible embodiment is that neither hydrogen nor the hydrogenation catalyst is present in step (a1).

The temperature in step (a1) may be up to 120°C and is preferably between 0°C and 100°C. More preferably the temperature is between 20-100°C.

It has been found that the best results with regard to the yield to primary amino compounds and $\varepsilon$-caprolactam are achieved when the conversion of the aldehyde compound in step (a1) is more than 90%, preferably more than 99%. Too low of a conversion will result in an increase of, for example, the 6-hydroxycaproate ester (or acid or amide) and/or secondary amino compound formation. Formation of these compounds will negatively influence the overal yield to $\varepsilon$-caprolactam.

As explained above, a too low contact or residence time in step (a1) will result in undesirable by-product formation. The optimal residence or contact time at which the conversion of the aldehyde starting compound is virtually completed, will depend on the reaction conditions, for example: temperature, concentration of reactants and method of mixing. Longer contact or residence times than needed to achieve the above conversion are possible. The optimal residence time or contact time can be easily determined by the man skilled in the art. Starting from the temperature and concentration range here described the residence or contact time will under normal mixing condition preferably be higher than 5 seconds. Preferably the residence or contact time will be below 2 minutes.

Step (a1) is carried out in the presence of ammonia, preferably a molar excess of ammonia is chosen such that the molar ratio ammonia:aldehyde compound is between 1:1 and 500:1 calculated on the starting amount of the aldehyde compound. Preferably this ratio is higher than 5:1. If this ratio is too low the $\varepsilon$-caprolactam yield is negatively influenced.

Water will be formed in step (a1) as a reaction product of the reaction between the aldehyde compound and ammonia. Preferably step (a1) and step (a2) is performed in the presence of at least 10 weight percent of water.

Preferably the molar ratio ammonia:aldehyde compound (aldehyde compound and its reaction products in step (a1) is between 3:1 and 25:1 more preferably between 5:1 and 15:1.

The water content of the reaction mixture in step (a1) is preferably between 15-60 wt.% and more preferably between 20-50 wt.%.

The concentration of the aldehyde compound or the concentration of the sum of aldehyde compound and its reaction products in step (a1) is generally between 1 and 50 wt.% and preferably between 10 and 35 wt.%. High yields to $\varepsilon$-caprolactam can be advantageously achieved at these higher concentrations.

The pressure in step (a1) is not critical. The pressure is generally equal or greater than the resulting equilibrium pressure of the liquid reaction mixture and the temperature employed.

Step (a1) can be carried in the presence of a catalyst, for example an acid ion exchanger or an acidic metal oxide catalyst, for example alumina or $TiO_2$. The conversion of the aldehyde starting compound in the first step also proceeds favorably in the absence of a catalyst. Because the overall yield to $\varepsilon$-caprolactam is not greatly influenced by the presence of a catalyst in the first step, such a catalyst is generally not used.

The process according to the invention can be performed batch wise or continuously. A large scale commercial process will preferably be performed continuously. For step (a1), it is important that the reactants are sufficiently contacted at a certain temperature during a specified period of time optionally in the presence of a catalyst as described above. Any manner of contacting will usually suffice. For example a tube reactor with or without, for example, internal baffling or packing or a static mixer is a possible contacting unit for step (a1). To control the temperature in step (a1) it may be advantageous to use cooling devices, for example cooled walls or a cooling spiral placed in the contacting unit.

The above described ratios and concentrations and their preferred values for step (a1) also apply for step (a2) unless otherwise mentioned. Moreover the composition of the aqueous reaction mixture obtained in step (a1) is by preference, directly and without substantial separation of any of the compounds of the mixture, used in step (a2). This is advantageous because it results in a more simple process.

The reaction product obtained in step (a1) is converted in step (a2), to $\varepsilon$-caprolactam and the primary amino compounds under hydrogenating conditions in the presence of ammonia.

The primary amino compounds are 6-aminocaproamide, 6-aminocaproate ester and 6-aminocaproic acid. Oligomers which may be formed in step (a) can in this invention also be considered as primary amino compounds and as precursors to $\varepsilon$-caprolactam. The oligomers are for the greater part dimers of 6-aminocaproic acid or dimers of 6-aminocaproamide. Trimers and higher oligomers may also be formed.

When the aldehyde compound according to formula (1) is 5-formylvalerate ester a mixture of $\varepsilon$-caprolactam, 6-aminocaproic acid, 6-aminocaproamide and none or a small amount of 6-aminocaproate ester and/or oligomers will be obtained in step (a2). The hydrolysis of the ester-group mainly takes place in step (a2). When the aldehyde compound is 5-formylvaleric acid a mixture of $\varepsilon$-caprolactam, 6-aminocaproic acid, possibly some 6-aminocaproamide and possibly some oligomers will be obtained in step (a2).

By 'hydrogenating conditions' is understood in this invention that the reaction conditions are such that the intermediate reaction product(s) obtained in step (a1) can be reduced by hydrogen. In general hydrogenation conditions are achieved when hydrogen and a hydrogenation catalyst are present. The hydrogenation catalyst is described above.

The total pressure used in step (a2) is generally between 0.5 and 20 MPa. The pressure is preferably between 0.5-10 MPa and more preferably between 1-5 MPa.

Step (a2) is in general carried out at a temperature higher than 40°C. In general the temperature will be lower than 200°C. To achieve optimal overall yields to ε-caprolactam the temperature is more preferably between 70 and 180°C. Most preferably the temperature is higher than 100°C because high yields to ε-caprolactam can be obtained.

The residence or contact time in step (a2) should be high enough to reduce virtually all the intermediate products formed in step (a1) to ε-caprolactam and primary amino compounds. Higher residence time or contact time will result in that more ε-caprolactam and possibly oligomers are formed. The residence or contact time is preferably between around a half minute to a couple of hours. When the process is carried out batch wise or in a continuously operated slurry reactor the contact or residence time respectively will generally be higher than the residence time when a continuously operated tube reactor is used.

Step (a2) may be performed continuously in a fixed bed reactor in which the heterogeneous hydrogenation catalyst is present. An advantage of this reactor is that the reactants are easily separated from the hydrogenation catalyst. Another manner of operating step (a2) is by way of one or more continuously operated well mixed contactors in series in which the hydrogenation catalyst is present as a slurry (slurry reactor). This manner of operation has the advantage that the heat of the reaction of step (a2) can be easily controlled by, for example, a cooled feed or by way of internally placed cooling devices. Examples of specific and suitable slurry reactors are one or multiple staged bubble columns or a gas lift-loop reactor or a continuously stirred tank reactor (CSTR). The slurry-hydrogenation catalyst can be separated from the reaction mixture after step (a2) by for example using hydrocyclones and/or by filtration, for example by cake- or cross-flow filtration.

The catalyst concentration in step (a2) may be choosen between a wide range. In a fixed bed reactor the amount of catalyst per volume will be high while in a slurry reactor this concentration will in general be lower. In a continuously operated slurry reactor the weight fraction of catalyst (including the carrier) is typically between 0.1 and 30 weight % relative to the total content of the reactor. The weight fraction will for example depend on the use of a carrier and the kind of carrier.

The yield to ε-caprolactam in step (a) is in the process according to the invention higher than 10 wt.% and preferably higher than 20 wt.%.

The high yields to ε-caprolactam can be achieved by for example increasing the concentration of the reactants in step (a), increasing the residence time in step (a) (or in step (a2) in the two step reductive amination) or by or increasing the temperature in step (a) (or in step (a2)).

Ammonia, hydrogen, the hydrogenation catalyst and the alcohol (if present) are preferably separated from the reaction mixture obtained in the reductive amination step (a) prior to the extraction according to the process of the invention. Hydrogen and part of the ammonia can be advantageously be separated by reducing the pressure and performing a gas/liquid separation. An example of such an operation is a flash operation performed at between ambient pressure and 0.5 MPa. The hydrogen and ammonia can advantageously be recycled to step (a).

In a subsequent step the alcohol (if present) can be separated. It has been found that it is advantageous to perform the cyclization step (b) in the pressence of less than 1 wt% and more preferably less than 0.1 wt% of alcohol. Thus when the resulting mixture of step (a) contains alcohol it is advantageous to separate this compound. It has been found that the pressence of alcohol during the cyclization promotes the formation of the corresponding N-alkyl caprolactam, an undesired by-product. The presence of small quantities of these N-alkylated products, for example N-methyl ε-caprolactam, in final ε-caprolactam makes the ε-caprolactam less suitable for use as starting material for nylon-6 fibers. Because these N-alkylated products are difficult to separate from the final ε-caprolactam, it is favorable that they do not form or that their formation is minimized in the process according to the invention.

Separating the alkanol may be performed by any known method known to the man skilled in the art, for example distillation or stripping, for example steam stripping.

Step (b) may be performed in the gas phase as described in for example US-A-4599199 or in US-A-3658810 by contacting a, preferably concentrated, mixture as obtained in step (a) with overheated steam having a temperature between 150-400°C at about atmospheric pressure. Such gas phase processes are advantageous because ε-caprolactam is obtained in a gaseous steam phase in which no oligomers are present. Separation of ε-caprolactam and oligomers can thus be avoided.

Preferably step (b) is performed in the liquid phase at super atmospheric pressures such as for example described in the aforementioned US-A-4730040, WO-A-9600722 and in the above mentioned article of Mares and Sheehan. High yields of ε-caprolactam of high quality can be obtained with these liquid phase processes. More preferably step (b) is performed in the liquid phase as discussed below.

The concentration of ammonia in step (b) is preferably below 5 wt.% and more preferably below 3 wt.% and most preferably below 1 wt.%. High concentrations of ammonia have a negative effect on the yield to ε-caprolactam per pass in a continuous process.

The concentration of ε-caprolactam and ε-caprolactam precursors in step (b) is preferably between 5 on 50 wt.%

and more preferably between 10-35 wt.%.

The elevated temperature in step (b) is between 200 and 350°C. Preferably the temperature in step (b) is higher than 290°C because of higher yield per pass to ε-caprolactam.

The pressure in step (b) is preferably between 5.0 and 20 MPa. Normally this pressure will be greater than or equal to the resulting pressure of the liquid reaction mixture and the temperature employed.

Step (b) can be performed continuously in process equipment resulting in high and low rates of backmixing.

The ε-caprolactam can be separated from the reaction mixture obtained in step (b) by for example crystallization, extraction or by distillation. Preferably ε-caprolactam is separated by extraction and more preferably by subjecting the effluent obtained in step (b) to the same extraction as subjected to the effluent of step (a) as described above. Prior to this extraction step it is preferred to separate part or all of the ammonia present in the aqueous mixture of step (b) in order to prevent a build up of ammonia in the process.

Extraction of ε-caprolactam from the effluent of step (b) is especially advantageous over distillation when oligomers are also present in the aqueous mixture containing the ε-caprolactam. When using distillation oligomers are usually obtained in the residue of the distillation(s) in a high concentration. Because of these high oligomer concentration fouling of, for example, pipes and other process equipment because of solidification of the oligomers can occur. This disadvantage does not occur when extraction is used as method for isolating ε-caprolactam.

Another advantage of extraction over distillation is that the amine compounds which can be present in the effluent of step (b) are not exposed to the high reboiler temperatures of the distillation. Under these high temperature conditions by-products and (more) oligomers tend to be formed. By using extraction as the method for isolating ε-caprolactam the exposure of the ε-caprolactam precursors to the high temperatures of the reboilers can be avoided. The ε-caprolactam may be purified by methods known for purifying ε-caprolactam obtained by Beckmann rearrangement. An example of a possible method of purifying ε-caprolactam is described in US-A-5496941.

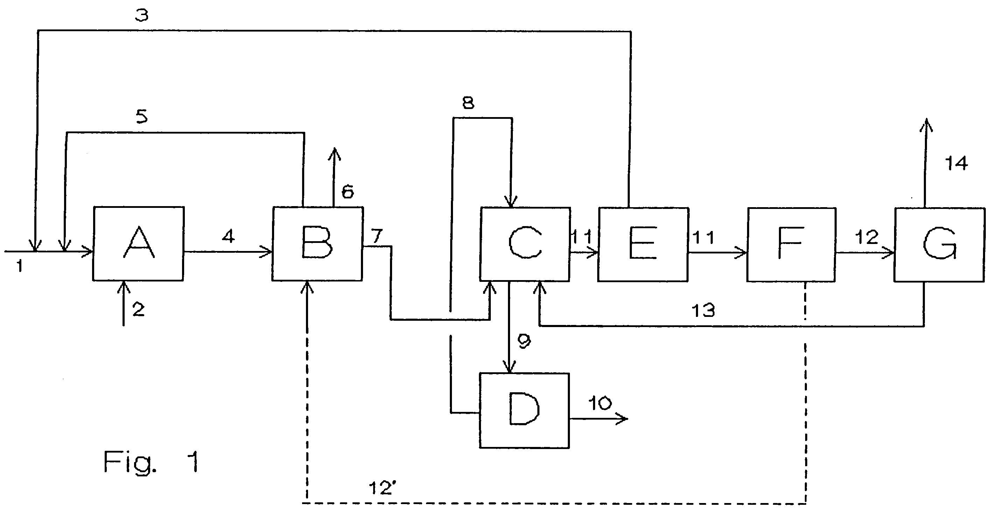

A non restrictive example of a possible embodiment of the process starting from methyl 5-formylvalerate according to the invention is given in Figure 1. The illustrated process is a schematic representation of the process equipment used in the below examples.

In figure 1 a mixture of methyl 5-formylvalerate/water/ammonia/methanol (1) is fed to the reductive amination reactor (A). Also fed to (A) is sufficient hydrogen (2). From the effluent of the reductive amination (4) ammonia and methanol is separated in (B) by steam stripping. Part of the methanol is recovered via (6) and the rest is recycled to the reductive amination via (5). The resulting reaction mixture (7) is extracted with the extraction solvent (8) in a counter current extraction column (C) yielding a stream rich in ε-caprolactam/extraction solvent (9) and an aqueous mixture (11) rich in 6-aminocaproic acid, 6-aminocaproamide and oligomers. First water is separated from this mixture in (E) by distillation and recycled to the reductive amination via (3). The resulting concentrated aqueous mixture is fed to the cyclization reactor (F) resulting in an effluent of the cyclization (12) rich in ε-caprolactam but also containing unconverted oligomers, 6-aminocaproic acid and 6-aminocaproamide. After separating ammonia (14) by steam stripping in (G) the aqueous mixture (13) is fed to the extraction column (C). Optionally the effluent of (F) is recycled to the steam stripper (B) via (12'). In this manner the feed of the extraction column is concentrated and ammonia is separated by using less process equipment. The ε-caprolactam extraction solvent mixture obtained in column (C) is supplied to a separation unit (D) in which the organic solvent is separated from the ε-caprolactam by for example distillation. The extraction solvent poor in ε-caprolactam thus obtained (8) is returned to column (C). In the recirculating streams purges will preferably be present (not shown) to overcome build up of contaminants and by-products.

The invention will be elucidated with the following non-restricting examples.

The composition of the resulting mixtures of the experiments are sometimes expressed in mol percentages. The molar percentage of a component is represented by the molar fraction (* 100%) of the molar amount of converted methyl 5-formylvalerate (M5FV) which contributes to that specific component. For example if the starting amount of M5FV is 100 mol and the resulting mixture contains 50 mol ε-caprolactam and 25 mol dimers then the molar contribution to ε-caprolactam will be 50 mol% and the molar contribution to the dimers will be 50 mol% (totaling 100 mol%). When no oligomers such as dimers are present in the mixture the above molar percentages are the same as the molar yield as expressed below:

$$\text{yield of component x} = \frac{\text{mol component x formed}}{\text{mol M5FV converted}} * 100\%$$

Example I

200 ml of a mixture of 20 wt.% ε-caprolactam and 5 wt.% 6-aminocaproic acid in water was well mixed long enough to reach equilibrium with 200 ml chloroform at room temperature and atmospheric pressure. The water phase was separated from the chloroform by phase separation. The water phase was again mixed with 200 ml chloroform. as above and separated from the chloroform by phase separation. The two chloroform phases were combined and analyzed by high pressure liquid gaschromatography (HPLC). The water phase was also analyzed and the partition coefficient (calculated as the concentration of $\varepsilon$-caprolactam in the organic chloroform phase divided by the concentration of $\varepsilon$-caprolactam in the aqueous phase at (almost) equilibrium conditions) was 0.74. No detectable amount of 6-aminocaproic acid (< 0.01 wt.%) was found in the chloroform phase.

Example II

Example I was repeated with dichloromethane. The partition coefficient was 0.84. No detectable amount of 6-aminocaproic acid was found in the dichloromethane phase.

Example III

Example I was repeated with methyl tert-butylether. The partition coefficient was 0.1. No detectable amount of 6-aminocaproic acid (< 0.01 wt.%) was found in the tert-butylether phase.

Example IV

Example I was repeated with a mixture as could be obtained in a step (a) of the present invention containing 5.08 wt.% $\varepsilon$-caprolactam, 3.09 wt.% 6-aminocaproic acid, 7.51 wt.% 6-aminocaproamide and 1.99 wt.% oligomers. The partition coefficient for $\varepsilon$-caprolactam was as in Example I. No detectable amounts (< 0.01 wt.%) of 6-aminocaproic acid, 6-aminocaproamide or oligomers were found in the organic phase.

Experiment V

Example IV was repeated at 80°C using the same volume of dodecylphenol instead of chloroform. The partition coefficient for $\varepsilon$-caprolactam was about 11.

Examples I-V illustrate that $\varepsilon$-caprolactam can be succesfully separated from aqueous mixtures containing 6-aminocaproic acid, 6-aminocaproamide and/or oligomers. These batch examples also show that an almost 100% separation of $\varepsilon$-caprolactam is possible in a continuously operated extraction; for example in a counter current extraction column or in a series of mixer/settlers.

Example VI

At a pressure of 3.0 MPa, 81.3 g/hr of methyl 5-formylvalerate 203 g/hr of ammonia and 526 g/hr of a 15 wt.% methanol in water mixture as pumped through a tube which was cooled by a water bath so that a constant temperature of 35°C was maintained in the tube. Almost no back-mixing occurred and the (liquid) residence time was 15 seconds.

The resulting mixture leaving the tube (step a1) was fed to a continuously stirred tank reactor, a Hastelloy C autoclave of 1 liter liquid volume. The reactor was stirred at 1250 rpm. The pressure was kept at a constant 3 MPa and the temperature at 120°C. To the reactor a net amount of 5 g/hr of hydrogen was fed. The reactor was filled with a 5 wt.% ruthenium on $Al_2O_3$ catalyst (Engelhard: ESCAT 44) and the catalyst concentration was kept at 96.0 g/l.

The composition of the effluent (mixture A) of the stirred reactor did not vary significantly during the 22 hours of operation. The average composition of all the products formed in the last 12 hours was 21.5 mol% 6-aminocaproic acid (6ACA), 45.9 mol% 6-aminocaproamide (6ACAM), 27.5 mol% $\varepsilon$-caprolactam (CAP), 2.1 mol % methyl 6-aminocaproate (M6AC) and 3.0 mol.% oligomers.

Mixture A was flashed to 0.1 MPa and continuously fed to the top of a steamstripper column (operated at 0.1 MPa) at a rate of 638g/hr. Steam was generated in a reboiler of the column. To the column also 212g/hr of fresh water was fed. The liquid bottom stream which left the steamstripper (rate 571 g/hr) did not contain any detectable amounts of methanol and ammonia. This aqueous stream consisted of 12.4 wt.% of 6ACA, 6ACAM, M6AC, CAP and oligomers having the same molar contribution as mixture A.

The aqueous mixture was subsequently fed to the bottom of a continuously operated counter current extraction column. To the top of this column (having 20 theoretical plates) chloroform was fed at a rate of 1.0 l/hr. $\varepsilon$-caprolactam was extracted to the chloroform phase with a more than 99% yield. Pratically all of the 6-aminocaproic acid, 6-aminocaproamide and oligomers remained in the aqueous phase.

This aqueous mixture was subsequently continuously fed to a cyclization reactor, a plugflow reactor (almost no backmixing), at a constant temperature of 320°C (maintained with the use of an oil bath), a pressure of 12 MPa and at a residence time of 30 minutes at a rate of 554 g/hr. The average composition of all the products present in the liquid aqueous stream leaving the cyclization reactor amounted to 7.5 wt.% caprolactam, 1.6 wt.% of 6-aminocaproic acid, 6-aminocaproamide and oligomers.

This aqueous mixture was fed to an extraction comparable as the one described earlier. The stream of chloroform leaving the extraction 41.6 g/hr $\varepsilon$-caprolactam. The total yield in this one pass was 92.7% calculated on the molar amount of methyl 5-formylvalerate.

By recycling the aqueous mixture obtained in the extraction to the cyclization reactor more $\varepsilon$-caprolactam can be obtained. It must be clear that the extract ions here illustrated can be combined in one unit operation in a commercially operated process.

**Claims**

1. Process to prepare $\varepsilon$-caprolactam, in which in a step (a) a compound with the general formula:

$$O=CH-(CH_2)_4-C(O)-R \qquad (1)$$

in which R is -OH, -NH$_2$ or O-R', in which R' is an organic group with 1 to 10 carbon atoms, is contacted with ammonia and hydrogen in a suitable solvent at elevated pressure in the presence of a hydrogenation catalyst to a mixture of primary amino compounds and $\varepsilon$-caprolactam, followed by a separate second step (b) in which the primary amino compounds are reacted to $\varepsilon$-caprolactam, characterized in that the solvent in step (a) is water or an aqueous medium, the yield to $\varepsilon$-caprolactam in step (a) is more than 10%, calculated on the initial molar amount of the compound according to formula (1), that $\varepsilon$-caprolactam is separated from the aqueous mixture obtained in step (a) by extraction using an organic extraction agent and that the aqueous mixture resulting from the extraction, containing primary the amino compounds, is used in step (b).

2. Process according to claim 1, characterized in that the extraction agent is a chlorinated hydrocarbon solvent.

3. Process according to claims 2, characterized in that the chlorinated hydrocarbon solvent is dichloromethane, chloroform or 1,1,1-trichloroethane.

4. Process according to claim 1, characterized in that the extraction agent is a phenol substituted with one or more alkyl groups.

5. Process according to any one of claims 1-4, characterized in that the starting compound according to formula (1) is an alkyl 5-formylvalerate compound, in which R' is a C$_1$-C$_6$ alkyl group, and that the starting aqueous reaction medium also contains between 2 and 20 weight% of the corresponding C$_1$-C$_6$ alkanol according to R'-OH.

6. Process according to any one of claims 1-5, characterized in that the $\varepsilon$-caprolactam is separated from the aqueous mixture obtained in step (b) and from the aqueous mixture obtained in step (a) by the same extraction.

7. Process according to any one of claims 1-6, characterized in that the process is performed continuously.

8. Process according to any one of claims 1-7, characterized in that step (a) is performed in two separate steps (a1) and (a2):

   (a1) reacting the compound according to formula (1) with ammonia under non-hydrogenation conditions and
   (a2) converting the reaction product obtained in step (a1) to $\varepsilon$-caprolactam and primary amino compounds under hydrogenation conditions in the presence of ammonia.

9. Process according to claim 8, characterized in that the hydrogenation conditions in step (a2) are achieved by performing step (a2) in the presence of hydrogen and a ruthenium containing catalyst.

10. Process according to any one of claims 1-9, characterized in that step (b) is performed in the liquid phase in which the concentration of ammonia is less than 3 wt.%, the concentration of $\varepsilon$-caprolactam and primary amino compounds is between 10-35 wt.% and the temperature is between 290 and 350°C.

Fig. 1

EP 0 826 666 A1

European Patent
Office

**EUROPEAN SEARCH REPORT**

Application Number

EP 96 20 2438

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | EP-A-0 234 295 (BASF AG) 2 September 1987<br>* column 5, line 1 - line 13; claims 1-3,5; examples 1,2 * | 1-10 | C07D201/00<br>C07D201/08 |
| Y | DE-A-36 02 376 (BASF AG) 30 July 1987<br>* column 4, line 63 - line 67; claims 1-3,5 * | 1-10 | |
| Y | DE-A-38 43 791 (BASF AG) 5 July 1990<br>* column 4, line 12 - line 33; claim 1 * | 1-10 | |
| Y | DE-A-36 02 375 (BASF AG) 30 July 1987<br>* column 4, line 67 - column 5, line 9; claim 1 * | 1-10 | |
| A | EP-A-0 242 505 (BASF AG) 28 October 1987<br>* claims 1-9 * | 1-10 | |
| A | DE-A-36 02 377 (BASF AG) 30 July 1987<br>* claims 1-9 * | 1-10 | |
| A | DE-A-17 95 013 (KANEGAFUCHI BOSEKI K. K.) 13 April 1972<br>* claims 1-9 * | 1-10 | TECHNICAL FIELDS SEARCHED (Int.Cl.6)<br><br>C07D |
| A | US-A-3 485 821 (TECHNI-CHEM CO.) 23 December 1969<br>* claims 1-15 * | 1-10 | |

The present search report has been drawn up for all claims

| Place of search | Date of completion of the search | Examiner |
|---|---|---|
| MUNICH | 10 January 1997 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document